# EUROPÄISCHE PATENTSCHRIFT

(11) **EP 3 551 242 B1**
(45) Veröffentlichungstag und Bekanntmachung des Hinweises auf die Patenterteilung: **13.01.2021**
(21) Anmeldenummer: 18729583.7
(22) Anmeldetag: 17.05.2018
(51) Int. Cl.: A61L 29/08, A61L 29/14, A61L 31/10, A61L 31/14, A61L 33/00, C08L 33/08, C08L 83/04

(54) **BESCHICHTUNG FÜR MEDIZINPRODUKTE**
COATING FOR MEDICINAL PRODUCTS
REVÊTEMENT POUR PRODUITS MÉDICAUX

(30) Priorität: 17.05.2017 DE 102017110748; 24.05.2017 DE 102017111486
(43) Veröffentlichungstag der Anmeldung: 16.10.2019
(73) Patentinhaber: Phenox GmbH, 44801 Bochum (DE)
(72) Erfinder: SCHEDLER, Uwe, 15366 Neuenhagen (DE); HEISE, Christian, 14776 Brandenburg (DE); THIELE, Thomas, 13351Berlin (DE); HENKES, Hans, 70192 Stuttgart (DE); MONSTADT, Hermann, 44797 Bochum (DE); HANNES, Ralf, 44137 Dortmund (DE); LENZ-HABIJAN, Tim, 44627 Herne (DE); BANNEWITZ, Catrin, 44789 Bochum (DE)
(74) Vertreter: Schneiders & Behrendt Bochum
(86) Internationale Anmeldenummer: PCT/EP2018/062848
(87) Internationale Veröffentlichungsnummer: WO 2018/210989

(56) Entgegenhaltungen:
- WO-A1-2008/006911
- US-A1- 2008 004 410

## Beschreibung

Die Erfindung betrifft Medizinprodukte mit einer Beschichtung, wobei die Beschichtung im Wesentlichen biomimetische und/oder biorepulsive Eigenschaften umfasst. Daneben betrifft die Erfindung auch ein Verfahren zur Beschichtung der Medizinprodukte.

Der Einsatz von Medizinprodukten zur Behandlung verschiedenster Krankheitsbilder nimmt stetig zu. Viele dieser Medizinprodukte gelangen während der Behandlung zumindest zeitweise oder auch dauerhaft in das Gefäßsystem oder allgemein in den Körper des Patienten und kommen so in direkten Kontakt mit dem Blut des Patienten. Dabei kommt es regelmäßig auch über das Blut und seine entsprechenden Bestandteile zu Reaktionen des Körpers, die in aller Regel unerwünscht sind, den Behandlungserfolg gefährden und zu teils lebensbedrohlichen Zuständen für den Patienten führen können.

Eine dieser unerwünschten Reaktionen betrifft die bei einem Patienten durch die bei der Behandlung verwendeten Medizinprodukte ausgelöste Blutgerinnung und im Speziellen die Thrombozytenadhäsion und die Thrombozytenaggregation. Die Thrombozytenadhäsion und die Thrombozytenaggregation und somit die Bildung von Blutgerinnseln, sogenannten Thromben, kann bei dauerhaften oder zeitweisen Implantaten ebenso beobachtet werden wie bei medizinischen Instrumenten, die nur kurzzeitig zur Behandlung oder zu Diagnosezwecken in den Körper des Patienten eingeführt werden. Die Thrombozyten haften auf der durch körpereigene Proteine markierten Oberfläche des eingeführten Medizinprodukts (Thrombozytenadhäsion), wodurch es in der Folge zur Bildung eines Thrombus kommen kann (Thrombozytenaggregation).

Löst sich solch ein Thrombus beispielsweise im Gefäßsystem, kann es zu teils schwerwiegenden bis hin zu tödlichen Folgeerkrankungen kommen. Dies ist vor allem dann der Fall, wenn der losgelöste Thrombus fortgespült wird und sich als Embolus in kleineren Gefäßen festsetzt, diese weitestgehend verschließt und so die ausreichende Versorgung der sich anschließenden Bereiche gefährdet. Resultierende Krankheitsbilder können beispielsweise Schlaganfälle, Herzinfarkte oder Thrombosen sein.

Um diese Risiken für den Patienten zu minimieren, wird heutzutage in der Regel im Rahmen einer Operation beziehungsweise Intervention auf die duale Plättchenhemmung zur Reduzierung des Thromboserisikos zurückgegriffen. Dabei erhält der Patient in der Regel vor dem Eingriff eine erste Dosis einer Kombination von Thrombozytenaggregationshemmern, insbesondere einer Kombination von Acetylsalicylsäure (ASS) und Clopidogrel, und muss diese auch nach dem Eingriff noch für eine bestimmte Zeit regelmäßig einnehmen. Daneben können auch andere Thrombozytenaggregationshemmer zum Einsatz kommen, die in den entsprechenden Leitlinien zur dualen Plättchenhemmung genannt sind. In der Regel werden diese anstelle des Clopidogrels in Kombination mit ASS verwendet. Beispiele sind Prasugel oder Ticagrelor.

Der Nachteil der dualen Plättchenhemmung liegt darin, dass sie systemisch erfolgt und somit auch systemisch wirkt. Für die Dauer der dualen Plättchenhemmung ist somit das Blutungsrisiko für den Patienten insgesamt erhöht.

Ein weiterer Nachteil besteht darin, dass die duale Plättchenhemmung für einige Patientengruppen gar nicht möglich ist, da für diese die mit der dualen Plättchenhemmung verbundenen Risiken, insbesondere das erhöhte Blutungsrisiko, so groß sind, dass diese die Vorteile von vornherein überwiegen. Im Extremfall stehen manchen Patienten bestimmte Behandlungsmethoden allein aufgrund der Tatsache nicht zur Verfügung, dass sie nach einem Eingriff nicht ausreichend gegen ein Thromboserisiko geschützt werden können. Die duale Plättchenhemmung ist beispielsweise Standard nach der Implantation endovaskulärer Prothesen (Stents und dergleichen).

Wünschenswert wäre es somit, wenn man ein Medizinprodukt zur Verfügung stellen könnte, das der Körper nicht als Fremdkörper erkennt oder zumindest keine für den Behandlungserfolg unerwünschten Reaktionen, wie beispielsweise Blutgerinnungsreaktionen, darauf zeigen würde und somit ein Verzicht auf die duale Plättchenhemmung bei solchen Medizinprodukten möglich wäre.

Aus der WO 2008/006911 A1 sind beschichtete Implantate wie z. B. Stents bekannt, wobei zunächst ein Primer und dann ein biokompatibles Polymer auf das Implantat aufgebracht werden. Anschließend erfolgt noch eine Quervernetzung der Polymerbeschichtung. Auf diese Weise werden verhältnismäßig dicke Beschichtungen erzeugt, die insbesondere dazu geeignet sind, eine kontrollierte Freisetzung von Wirkstoffen zu bewirken.

Aufgabe der Erfindung ist es, ein Medizinprodukt zur Verfügung zu stellen, das die angesprochenen Nachteile im Wesentlichen nicht aufweist.

Weiterhin ist es Aufgabe der Erfindung, eine Beschichtung für Medizinprodukte bereitzustellen, die der Oberfläche von Medizinprodukten insbesondere biomimetische und/oder biorepulsive Eigenschaften verleiht und Thrombozytenreaktionen, insbesondere Thrombozytenadhäsion, weitestgehend nicht auslöst.

Gelöst wird diese Aufgabe durch ein Medizinprodukt umfassend mindestens ein Substrat mit einer Beschichtung, wobei die Beschichtung eine Funktionsschicht umfasst und die Funktionsschicht mindestens ein Monosaccharid umfasst, wobei die Monosaccharide durch eine Funktionalisierung der Monosaccharide direkt oder indirekt an das Substrat bindbar sind und erst bei der Bindung an das Substrat oligo- oder polymerisieren, wobei die Monosaccharide in ihrer nicht funktionalisierten Form einen Zuckeralkohol oder eine hiervon durch Dehydratisierung ableitbare cyclische oder heterocyclische Verbindung umfassen. Daneben betrifft die Erfindung ein entsprechendes Verfahren.

Vorteilhafte Ausgestaltungen sind jeweils Gegenstand der abhängigen Ansprüche.

Ein erfindungsgemäßes Medizinprodukt umfasst im Wesentlichen zumindest ein Substrat und eine Funktionsschicht. Die Funktionsschicht weist bevorzugt biomimetische und/oder biorepulsive Eigenschaften auf.

Seit vielen Jahren bekannt sind medikamentenbeschichtete Implantate, welche einen anderen Ansatz zur Vermeidung von Abwehrreaktionen des Körpers verfolgen. Insbesondere sind hier anti-proliferative medikamentenbeschichtete Stents zu nennen, die im Gegensatz zur hier beschriebenen Erfindung die neointimale Hyperplasie reduzieren.

Ebenfalls bekannt ist aus der US 2008/0004410 A1 das Aufbringen von Polymeren auf medizinische Vorrichtungen wie Kontaktlinsen, um auf diese Weise eine hydrophile oder die Gleiteigenschaften verbessernde Beschichtung zu erzeugen.

Gemäß der vorliegenden Erfindung befindet sich auf dem Substrat selbst zumeist noch eine Trägerschicht, die Haftvermittler umfasst, über die die Funktionsschicht mit dem Substrat verbindbar ist. Bevorzugte Haftvermittler im Sinne der Erfindung sind Silanhaftvermittler. Alternativ können auch andere Haftvermittler, beispielsweise polyolefinische Haftvermittler oder Haftvermittler auf Basis von Titanaten oder Zirkonaten zum Einsatz kommen.

Weitere Beispiele für Haftvermittler sind
- Thiole und Dithioverbindungen, besonders geeignet für Edelmetallsubstrate
- Amine und Alkohole, besonders beeignet für Platinsubstrate
- Carbonsäuren, besonders geeignet für Silbersubstrate und Aluminiumsubstrate, wobei das Aluminium eine Aluminiumoxidoberfläche aufweisen kann
- Phosphonsäuren (Phosphonate), besonders geeignet für Eisen-, Eisenoxid-, Titan- und Titandioxidsubstrate
- Komplexbildende Haftvermittler, insbesondere Chelate, die zum Teil auch nicht-kovalent an Substrate binden, besonders geeignet für verschiedene Metall- und Metalloxidsubstrate

In allen Fällen sollten die Haftvermittler über funktionelle Gruppen verfügen, über die eine Reaktion des Haftvermittlers mit der Funktionsschicht und damit eine in der Regel kovalente Ankopplung ermöglicht wird.

Vorzugsweise ist das Substrat geeignet, mit einem Haftvermittler eine Bindung einzugehen. Solche Substrate sollen im Sinne dieser Anmeldung "beschichtbares Substrat" genannt werden. Beschichtbare Substrate umfassen entsprechend solche Substrate, deren Oberfläche ausreichend reaktiv und/oder ausreichend aktivierbar ist, um zumindest teilweise mit einem Haftvermittler oder auch direkt mit der Funktionsschicht Bindungen einzugehen. Vorzugsweise umfassen die Bindungen zwischen Substrat und Haftvermittler kovalente Bindungen.

Beschichtbare Substrate im Sinne der Erfindung können entsprechend verschiedenste, insbesondere oxidierbare Substrate und Kombinationen daraus sein. Darunter fallen beispielsweise Metalle wie Nickel, Titan, Platin, Iridium, Gold, Kobalt, Chrom, Aluminium, Eisen oder Legierungen sowie Kombinationen hiervon. Beispielsweise kann auch ein Metall mit einem anderen Metall beschichtet werden, wobei auf die äußere Metallschicht wiederum die Trägerschicht und die Funktionsschicht aufgebracht werden. Unter beschichtbaren Metallen werden auch solche Substrate verstanden, bei denen das eigentliche Metall von einer Oxidschicht bedeckt ist. Weitere beschichtbare Substrate sind Gläser.

Beschichtbare Substrate im Sinne der Erfindung können auch verschiedenste Kunststoffe sein, wie beispielsweise Polyamide (PA), Polytetrafluorethylen (PTFE), expanded Polytetrafluorethylen (ePTFE), Polylactide (PLA), Polyester, Polyether, Polyurethan, Polyolefine, weiterhin entsprechende BlockCopolymere. Dem Fachmann ist eine Vielzahl an geeigneten Kunststoffen im Bereich der Medizintechnik bekannt. Während bei metallischen oder oxidischen Oberflächen in der Regel ein Haftvermittler erforderlich ist, kommt ein solcher bei Polymeren als Substrat nicht immer zum Einsatz.

Eine geeignete Haftvermittlung kann beispielsweise über eine Silanisierung erfolgen, also eine chemische Anbindung von Silicium-, insbesondere Silanverbindungen an zumindest Teilen ihrer Oberfläche. An Oberflächen binden Silicium- und Silanverbindungen beispielsweise an Hydroxy- und Carboxy-Gruppen.

Auch Polyolefine können als Haftvermittler eingesetzt werden, darunter chlorierte Polyolefine (CPO) oder acrylierte Polyolefine (APO).

Die Erfindung ist jedoch nicht auf Beschichtungen der genannten Kunststoffe und Metalle beschränkt, vielmehr sind diese nur beispielhaft genannt. Prinzipiell richtet sich die Erfindung auf Beschichtungen aller denkbaren Materialien, die ein beschichtbares Substrat im Sinne der Erfindung darstellen.

Unter einer Silanverbindung im Sinne der Erfindung sind all jene Verbindungen zu verstehen, die der allgemeinen Formel RₘSiXₙ folgen (m, n = 0-4, wobei R für organische Reste, insbesondere Alkyl-, Alkenyl- oder Arylgruppen, und X für hydrolysierbare Gruppen, insbesondere OR, OH oder Halogen mit R = Alkyl, Alkenyl oder Aryl steht. Insbesondere kann das Silan die allgemeine Formel RSiX₃ haben. Darüber hinaus gehören entsprechende Verbindungen mit mehreren Siliciumatomen zu den Silanverbindungen im Sinne der Erfindung. Insbesondere Silan-Derivate in Form von siciliumorganischen Verbindungen werden als Silanverbindungen im Sinne der Erfindung aufgefasst. Unter Silanverbindungen im Sinne der Erfindung sind entsprechend nicht nur solche Stoffe zu verstehen, die aus einem Silicium-Grundgerüst und Wasserstoff bestehen und die Bezeichnung Silane tragen.

Die Matrix der Funktionsschicht ist bevorzugt kovalent an die Trägerschicht oder das Substrat gebunden und wird bevorzugt mittels Pfropfpolymerisation synthetisiert, wobei die Funktionsschicht auf der Trägerschicht bzw. dem Substrat erzeugt wird. Die Polymerisation der aufgebrachten Monosaccharide, wobei Zuckeralkohole (Alditole) als solche aufgefasst werden, erfolgt im Wesentlichen erst auf der Trägerschicht/dem Substrat beziehungsweise innerhalb der Funktionsschicht.

Es ist für die Erfindung unerheblich, in welcher Form die (Pfropf)polymerisation abläuft. Insbesondere kann ausgehend von einer Hauptkette das Wachstum der Seitenketten starten. Dieser Ansatz wird auch als "grafting from" bezeichnet. Ebenso ist es möglich, dass die Seitenketten bereits mit der Oligo- oder Polymerisation begonnen haben und die bereits wachsenden Seitenketten sich mit der Hauptkette verbinden ("grafting onto"). Schließlich können sich auch bereits oligo- oder polymerisierte Haupt- und Seitenketten zusammenfinden ("grafting through").

Die Funktionsschicht umfasst bevorzugt im Wesentlichen eine komplexe, stark verzweigte, hydrophile Matrix umfassend eine Vielzahl von Molekülen mit jeweils einer Hauptkette als polymerem Rückgrat und jeweils mehreren Seitenketten. Die Haupt- und/oder Seitenketten können mit weiteren Haupt- und/oder Seitenketten Bindungen eingehen. Weitere matrixbildende Mono-, Oligo- und Polymere können in diese Haupt- und Seitenketten eingebunden sein, ohne selbst kovalent an die Trägerschicht gebunden zu sein.

Die Hauptkette kann zumindest teilweise polymerisierte Vinyl-, Allyl-, Acryl- oder Methacrylverbindungen oder deren Derivate und/oder deren Isomere oder auch Kombinationen davon umfassen.

Die Seitenketten umfassen Monosaccharide, wobei auch Zuckeralkohole (Alditole) als solche aufgefasst werden.

Das erfindungsgemäße Medizinprodukt umfasst mindestens ein Substrat mit einer Beschichtung, wobei die Beschichtung bevorzugt eine auf dem Substrat befindliche Trägerschicht und eine auf der Trägerschicht befindliche Funktionsschicht umfasst. Die Trägerschicht umfasst im Wesentlichen die Haftvermittler, die zumeist kovalent an das Substrat gebunden sind. Daneben sind auch nicht kovalent bindende Haftvermittler bekannt, beispielsweise solche, die über eine Komplexbindung an das Substrat binden. Bevorzugte Haftvermittler sind Siliciumverbindungen und polyolefinische Haftvermittler. Die Funktionsschicht umfasst gemäß einer bevorzugten Ausführungsform mindestens einen funktionalisierten Zuckeralkohol, über den die Funktionsschicht kovalent an die Trägerschicht gebunden ist.

Ein bevorzugter Zuckeralkohol der Funktionsschicht entspricht in seiner nicht funktionalisierten Form einem Zuckeralkohol mit der Summenformel C₆H₁₄O₆, beispielsweise Sorbitol, und/oder dessen Derivate, wie beispielsweise Sorbitan, und/oder dessen Isomere, wie beispielsweise Mannitol.

"In seiner nicht funktionalisierten Form" bedeutet, dass die angegebene Summenformel die Summenformel des nicht funktionalisierten Zuckeralkohols darstellt. Unter Funktionalisierung wird dabei die Einführung einer Funktion in die Verbindung verstanden, die eine Verknüpfung mit dem Substrat, der Trägerschicht und/oder bereits zuvor mit der Trägerschicht oder dem Substrat verbundenen Verbindungen erlaubt.

Es ist dem Fachmann klar, dass die erfindungsgemäße Funktionsschicht auch funktionalisierte Varianten des Zuckeralkohols mit der Summenformel C₆H₁₄O₆ umfasst. Insbesondere ist dem Fachmann klar, dass die Funktionsschicht eine komplexe Matrix umfasst, die durch die Polymerisation der aufgebrachten, funktionalisierten Zuckeralkohole entstehen kann.

Als Derivate sind im Sinne der Erfindung alle von dem Stoff durch Dehydratisierung ableitbaren cyclischen und heterocyclischen Verbindungen zu verstehen.

Bevorzugt ist der Zuckeralkohol beziehungsweise sind die Zuckeralkohole über mindestens eine reaktive Gruppe funktionalisiert, wobei die reaktive Gruppe vorzugsweise eine reaktive Mehrfach-, insbesondere Doppelbindung umfasst und wobei diese reaktive Doppelbindung vorzugsweise eine Acryl-Gruppe ist. Andere für die Polymerisierung geeignete Funktionsgruppen, die nicht unbedingt eine reaktive Doppelbindung aufweisen müssen, sind dem Fachmann bekannt und umfassen beispielsweise Methacrylgruppen, Vinylgruppen oder auch Allylgruppen.

Die Zuckeralkohole der Funktionsschicht sind zumindest teilweise untereinander oligo- oder polymerisiert.

Das Medizinprodukt umfasst mindestens ein Substrat mit einer Beschichtung, wobei die Beschichtung eine Funktionsschicht umfasst. Die Funktionsschicht umfasst mindestens ein funktionalisiertes Monosaccharid, wobei die Monosaccharide an die Trägerschicht bindbar sind und erst bei der Bindung an die Trägerschicht oligo- oder polymerisieren.

Vorzugsweise umfasst die Beschichtung eine auf dem Substrat befindliche Trägerschicht, wobei die Funktionsschicht wiederum an die Trägerschicht gebunden ist. Bei den ausgebildeten Bindungen kann es sich insbesondere um kovalente, ggf. aber auch um andere Bindungen wie Komplexbindungen handeln. Die Trägerschicht umfasst im Wesentlichen die Haftvermittler, die an das Substrat gebunden sind. Bevorzugte Haftvermittler sind Siliciumverbindungen und polyolefinische Haftvermittler.

Das Monosaccharid der Funktionsschicht umfasst mindestens einen Zuckeralkohol und/oder dessen oben definierte Derivate.

Ein bevorzugter Zuckeralkohol der Funktionsschicht entspricht in seiner nicht funktionalisierten Form einem Zuckeralkohol mit der Summenformel C₆H₁₄O₆, beispielsweise Sorbitol, und/oder dessen Derivate, beispielsweise Sorbitan, und/oder dessen Isomere, beispielsweise Mannitol. Die Struktur von Sorbitol wird nachfolgend wiedergegeben:

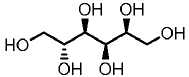

"In seiner nicht funktionalisierten Form" soll bedeuten, dass die angegebene Summenformel die Summenformel des nicht funktionalisierten Zuckeralkohols darstellt, jedoch gegebenenfalls auch dessen Derivate wie oben definiert umfassen soll.

Es ist dem Fachmann klar, dass die erfindungsgemäße Funktionsschicht funktionalisierte Varianten des Zuckeralkohols mit der Summenformel C₆H₁₄O₆ und/oder dessen Derivate wie oben definiert umfasst. Insbesondere ist dem Fachmann klar, dass die Funktionsschicht eine komplexe Matrix umfasst, die durch die Polymerisation der aufgebrachten, funktionalisierten Monosaccharide entstanden ist.

Die Monosaccharide der Funktionsschicht sind zumindest teilweise untereinander oligo- oder polymerisierbar.

Bevorzugt ist das Monosaccharid über mindestens eine reaktive Gruppe funktionalisiert, wobei die reaktive Gruppe vorzugsweise eine reaktive Mehrfachbindung, insbesondere Doppelbindung umfasst und wobei diese reaktive Doppelbindung vorzugsweise eine Acryl-Gruppe ist. Andere für die Polymerisierung geeignete funktionelle Gruppen, die nicht unbedingt eine reaktive Doppelbindung aufweisen müssen, sind dem Fachmann bekannt und umfassen beispielsweise Methacrylgruppen, Vinylgruppen oder auch Allylgruppen.

Die Lösung, aus der die Funktionsschicht der erfindungsgemäßen Beschichtung aufgebaut wird, kann demnach einzelne oder mehrere der nachfolgenden Stoffe umfassen:
(1) Sorbitol-Acrylate (mit einer oder mehreren Acrylatgruppe/n), wobei sich die Acrylatgruppe/n an verschiedenen Positionen befinden können. 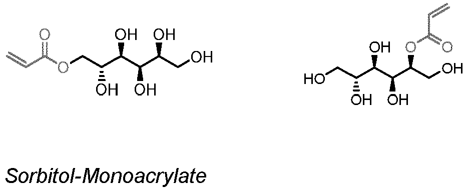 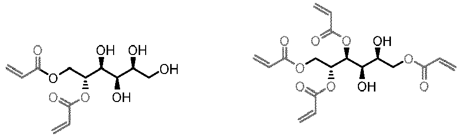 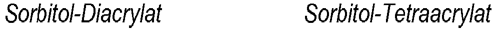
(2) Sorbitol-Acrylate (mit einer oder mehreren Acrylatgruppe/n), wobei die Sorbitol-Acrylate teilweise oxidiert sein können und eine Aldehyd-, Keto- und/oder eine Carboxygruppe umfassen können. 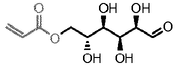 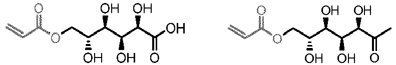
(3) Sorbitol-Acrylate (mit einer oder mehreren Acrylatgruppe/n), wobei diese weitere reaktive Gruppen umfassen können, beispielsweise Carboxygruppen. 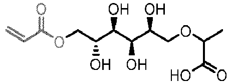
(4) Anhydride, beispielsweise Sorbitan-(Mono)-Acrylat mit einer polymerisierbaren Gruppe. 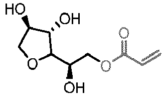
(5) Sorbitol mit einer nicht polymerisierbaren Gruppe, beispielsweise einer Carboxygruppe. 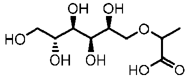
(6) Komplexe Sorbitol Verbindungen, die zwar nicht polymerisierbar sind, jedoch in die Polymermatrix der Funktionsschicht eingebaut werden können.

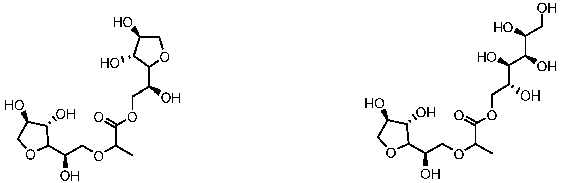

Die Struktur der Funktionsschicht kann über die konkrete Zusammensetzung der Stoffe variiert werden. So ist es beispielsweise möglich, engmaschigere Funktionsschichten durch einen erhöhten Anteil an Vernetzern zu erzeugen oder eher gering vernetzte Funktionsschichten mit längeren linearen Bereichen durch einen geringeren Anteil an Vernetzern.

Ohne sich an eine bestimmte Theorie binden zu wollen, wird der Vorteil der erfindungsgemäßen Beschichtung darin gesehen, dass die Funktionsschicht biomimetische bzw. biorepulsive Eigenschaften aufweist und von Thrombozyten nicht als körperfremd, sondern vielmehr als körpereigen erkannt wird. Entsprechend löst die erfindungsgemäße Funktionsschicht keine Reaktion der Thrombozyten aus, insbesondere keine Adhäsions- und auch keine Aggregationsreaktionen.

Der biomimetische Effekt der erfindungsgemäßen Beschichtung wird darauf zurückgeführt, dass die erfindungsgemäße Funktionsschicht die menschliche Glykokalyx nachahmt. Die Glykokalyx überzieht die Zellen der Blutgefäße mit einer Art Schleimschicht und besteht aus verschiedenen Polysacchariden, die kovalent an die Membranproteine (Glycoproteine) und Membranlipide (Glycolipide) gebunden sind.

Von Vorteil für den hohen biomimetischen Effekt der erfindungsgemäßen Beschichtung - und insbesondere der Funktionsschicht - ist, dass die Polymerisation der Reaktanten der Funktionsschichtlösung im Wesentlichen erst nach dem Auftragen der Funktionsschichtlösung auf das Substrat bzw. die Trägerschicht stattfindet. Hierdurch entsteht durch Polymerisation der Reaktanten eine komplexe Schicht, die eine so hohe Ähnlichkeit zur Glykokalyx aufweist, dass die Anhaftung von Thrombozyten an mit der erfindungsgemäßen Beschichtung versehenen Flächen deutlich geringer war als an unbeschichteten Oberflächen.

Der biorepulsive Effekt der erfindungsgemäßen Beschichtung wird auf das Prinzip der sterischen Repulsion zurückgeführt. Vermutlich wird der Raum, der den Oligo- und Polymeren auf der Oberfläche zur Verfügung steht, bei Annäherung eines Proteins reduziert, d. h. ein sich näherndes Protein zwingt die Oligo- und Polymere auf der Oberfläche, eine energetisch ungünstigere Konformation einzunehmen. Hierdurch ergibt sich insgesamt eine gegenüber Proteinen abstoßende Kraft. Möglicherweise führt auch die Verdrängung von Wassermolekülen aus der Beschichtung zu einer repulsiven osmotischen Kraft gegenüber Proteinen.

Für die Thrombozytenadhäsion bedeutet dieses Wirkungsprinzip, dass Thrombozyten nicht haften können, weil keine oder nur wenige zur Anbindung geeignete Proteine auf der Oberfläche vorhanden sind, wodurch die Thrombozytenadhäsion deutlich reduziert ist.

Vorteilhaft an der erfindungsgemäßen Beschichtung ist auch, dass die Beschichtung über den Zwischenschritt der Haftvermittlung nur solche Oberflächen und Strukturen des Medizinproduktes überzieht, die für die entsprechenden Haftvermittler aktivierbar sind und insbesondere auch aktiviert wurden. So ist es beim Aufbringen der Funktionsschichtlösung möglich, das komplette Medizinprodukt in die Funktionsschichtlösung einzubringen, ohne dass Bereiche zusätzlich geschützt werden müssten, die nicht beschichtet werden sollen.

Eine solche selektive Beschichtung bzw. ein in dieser Weise selektives Beschichtungsverfahren hat den zuvor beschriebenen Vorteil bei einer Vielzahl von Medizinprodukten, zumindest bei solchen, die verschiedene Materialien umfassen, eine Beschichtung jedoch nur auf bestimmten dieser Materialien erfolgen soll.

Die erfindungsgemäße Beschichtung lässt beim Beschichtungsprozess Möglichkeiten dafür, nur die Teile des Medizinprodukts zu aktivieren, die später auch die Funktionsschicht tragen sollen. Auch ist es denkbar, dass das Medizinprodukt bereits so konzipiert ist, dass für die zu beschichtenden Teile solche Stoffe gewählt werden, die aktivierbar für eine Haftvermittlung sind.

Medizinprodukte mit einer erfindungsgemäßen Beschichtung sind insbesondere für den endovaskulären, den neurovaskulären Einsatz und den kardiovaskulären Einsatz einsetzbar, jedoch kann die erfindungsgemäße Beschichtung für ein Medizinprodukt grundsätzlich immer dann sinnvoll sein, wenn das entsprechende Medizinprodukt mit Blut in Kontakt kommt.

### Versuche

Es wurden *in vitro* Versuchsreihen mit der erfindungsgemäßen Beschichtung durchgeführt, um die Effektivität der erfindungsgemäßen Beschichtung zu testen. Hierzu wurden je Versuchsreihe ein unbeschichtetes Nitinol-Plättchen und ein erfindungsgemäß silanisiertes und anschließend mit polymerisiertem Sorbitolacrylat beschichtetes Nitinol-Plättchen für 10 Minuten mit heparinisiertem Vollblut inkubiert. Die Adhäsion von Thrombozyten wurde anschließend mittels Fluoreszenzmikroskopie über fluoreszenzmarkierte CD61 Antikörper bestimmt.

Es zeigte sich eine deutlich geringere Adhäsion von Thrombozyten an den erfindungsgemäß beschichteten Nitinol-Plättchen gegenüber den unbeschichteten Nitinol-Plättchen.

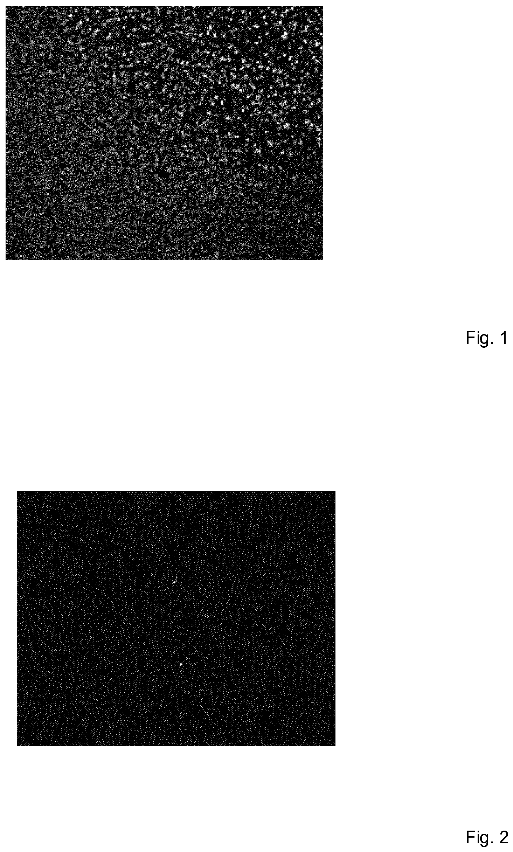

Fig. 1 zeigt ein unbeschichtetes Nitinolplättchen nach 10 Minuten Inkubationszeit mit heparinisiertem Vollblut bei 10x Vergrößerung unter dem Fluoreszenzmikroskop. Es ist deutlich die Adhäsion einer Vielzahl von CD61 positiven Thrombozyten zu erkennen.
Fig. 2 zeigt ein beschichtetes Nitinolplättchen nach 10 Minuten Inkubationszeit mit heparinisiertem Vollblut bei 10x Vergrößerung unter dem Fluoreszenzmikroskop. Es sind nur wenige angeheftete CD61 positive Thrombozyten zu erkennen.

## Patentansprüche

1. Medizinprodukt umfassend mindestens ein Substrat mit einer Beschichtung, wobei die Beschichtung eine Funktionsschicht umfasst und die Funktionsschicht mindestens ein Monosaccharid umfasst, **dadurch gekennzeichnet, dass** die Monosaccharide durch eine Funktionalisierung der Monosaccharide direkt oder indirekt an das Substrat bindbar sind und erst bei der Bindung an das Substrat oligo- oder polymerisieren, wobei die Monosaccharide in ihrer nicht funktionalisierten Form einen Zuckeralkohol oder eine hiervon durch Dehydratisierung ableitbare cyclische oder heterocyclische Verbindung umfassen.

2. Medizinprodukt nach Anspruch 1, **dadurch gekennzeichnet, dass** die Beschichtung eine auf dem Substrat befindliche Trägerschicht mit einem Haftvermittler umfasst und die Funktionsschicht an die Trägerschicht gebunden ist.

3. Medizinprodukt nach Anspruch 2, **dadurch gekennzeichnet, dass** die Bindung des Haftvermittlers an das Substrat und/oder die Bindung der Funktionsschicht an die Trägerschicht eine kovalente Bindung ist.

4. Medizinprodukt nach Anspruch 2 oder 3, **dadurch gekennzeichnet, dass** der Haftvermittler eine Siliciumverbindung, insbesondere eine Silanverbindung, umfasst.

5. Medizinprodukt nach einem der Ansprüche 1 bis 4, **dadurch gekennzeichnet, dass** der Zuckeralkohol der Funktionsschicht in seiner nicht funktionalisierten oder dehydratisierten Form mindestens einen Zuckeralkohol der Summenformel C₆H₁₄O₆ umfasst.

6. Medizinprodukt nach einem der Ansprüche 1 bis 5, **dadurch gekennzeichnet, dass** das Monosaccharid der Funktionsschicht in nicht an die Trägerschicht oder das Substrat gebundener Form über mindestens eine reaktive Mehrfachbindung, insbesondere Doppelbindung funktionalisiert ist.

7. Medizinprodukt nach Anspruch 6, **dadurch gekennzeichnet, dass** die reaktive Doppelbindung Bestandteil einer (Meth)acryl-Gruppe ist.

8. Medizinprodukt nach einem der vorherigen Ansprüche, **dadurch gekennzeichnet, dass** das Substrat mindestens ein Metall umfasst, ausgewählt aus der Gruppe umfassend Nickel, Titan, Platin, Iridium, Gold, Kobalt, Chrom, Aluminium, Eisen und/oder eine Legierung davon.

9. Medizinprodukt nach einem der Ansprüche 1 bis 7, **dadurch gekennzeichnet, dass** das Substrat mindestens einen Kunststoff umfasst, ausgewählt aus der Gruppe umfassend Polyamide (PA), Polytetrafluorethylen (PTFE), expanded Polytetrafluorethylen (ePTFE), Polylactide (PLA), Polyester, Polyether, Polyurethan, Polyolefine, weiterhin entsprechende BlockCopolymere.

10. Medizinprodukt nach einem der vorherigen Ansprüche, **dadurch gekennzeichnet, dass** es sich um ein Medizinprodukt für den endovaskulären Einsatz handelt.

11. Medizinprodukt nach einem der Ansprüche 1 bis 9, **dadurch gekennzeichnet, dass** es sich um ein Medizinprodukt für den neurovaskulären Einsatz handelt.

12. Medizinprodukt nach einem der Ansprüche 1 bis 9, **dadurch gekennzeichnet, dass** es sich um ein Medizinprodukt für den kardiovaskulären Einsatz handelt.

13. Verfahren zur Beschichtung eines ein Substrat aufweisenden Medizinprodukts mit einer eine Funktionsschicht aufweisenden Beschichtung, die die Adhäsion von Thrombozyten verringert, wobei die Funktionsschicht mindestens ein Monosaccharid umfasst und die Monosaccharide durch eine Funktionalisierung der Monosaccharide direkt oder indirekt an das Substrat gebunden werden und erst bei der Bindung an das Substrat oligo- oder polymerisieren, wobei die Monosaccharide in ihrer nicht funktionalisierten Form einen Zuckeralkohol oder eine hiervon durch Dehydratisierung ableitbare cyclische oder heterocyclische Verbindung umfassen.

## Claims

1. A medical device comprising at least one substrate having a coating, wherein the coating comprises a functional layer and the functional layer comprises at least one monosaccharide,
**characterised in that**
the monosaccharides can be bonded directly or indirectly to the substrate by a functionalisation of the monosaccharides, and they oligomerise or polymerise only upon bonding to the substrate, wherein the monosaccharides in their non-functionalised form comprise a sugar alcohol or a cyclic or heterocyclic compound that can be derived therefrom by dehydration.

2. The medical device according to claim 1, **characterised in that** the coating comprises a carrier layer which is located on the substrate and has an adhesion promoter, and the functional layer is bonded to the carrier layer.

3. The medical device according to claim 2, **characterised in that** the bond of the adhesion promoter to the substrate and/or the bond of the functional layer to the carrier layer is a covalent bond.

4. The medical device according to claim 2 or 3, **characterised in that** the adhesion promoter comprises a silicon compound, in particular a silane compound.

5. The medical device according to one of claims 1 to 4, **characterised in that** the sugar alcohol of the functional layer, in its non-functionalised or dehydrated form, comprises at least one sugar alcohol of the molecular formula C₆H₁₄O₆.

6. The medical device according to one of claims 1 to 5, **characterised in that** the monosaccharide of the functional layer, in a form in which it is not bonded to the carrier layer or the substrate, is functionalised via at least one reactive multiple bond, in particular a double bond.

7. The medical device according to claim 6, **characterised in that** the reactive double bond is a constituent of a (meth)acrylic group.

8. The medical device according to one of the preceding claims, **characterised in that** the substrate comprises at least one metal selected from the group comprising nickel, titanium, platinum, iridium, gold, cobalt, chromium, aluminium, iron and/or an alloy thereof.

9. The medical device according to one of claims 1 to 7, **characterised in that** the substrate comprises at least one plastic material selected from the group comprising polyamides (PA), polytetrafluoroethylene (PTFE), expanded polytetrafluoroethylene (ePTFE), polylactides (PLA), polyester, polyether, polyurethane, polyolefins, as well as relevant block copolymers.

10. The medical device according to one of the preceding claims, **characterised in that** it is a medical device for endovascular applications.

11. The medical device according to one of claims 1 to 9, **characterised in that** it is a medical device for neurovascular applications.

12. The medical device according to one of claims 1 to 9, **characterised in that** it is a medical device for cardiovascular applications.

13. A method for coating a medical device having a substrate with a coating having a functional layer, which reduces the adhesion of thrombocytes, wherein the functional layer comprises at least one monosaccharide and the monosaccharides are bonded directly or indirectly to the substrate via a functionalisation of the monosaccharides and they oligomerise or polymerise only upon bonding to the substrate, wherein the monosaccharides in their non-functionalised form comprise a sugar alcohol or a cyclic or heterocyclic compound that can be derived therefrom by dehydration.

## Revendications

1. Produit médical comprenant au moins un substrat doté d'un revêtement, le revêtement comprenant une couche fonctionnelle et la couche fonctionnelle comprenant au moins un monosaccharide,
**caractérisé en ce que**
les monosaccharides peuvent être liés directement ou indirectement au substrat par une fonctionnalisation des monosaccharides et oligomérisent ou polymérisent seulement lors de la liaison au substrat, les monosaccharides comprenant, sous leur forme non fonctionnalisée, un alcool glucidique ou un composé cyclique ou hétérocyclique pouvant être dérivé de celui-ci par déshydratation.

2. Produit médical selon la revendication 1, **caractérisé en ce que** le revêtement comprend une couche de support située sur le substrat dotée d'un promoteur d'adhérence et la couche fonctionnelle est liée à la couche de support.

3. Produit médical selon la revendication 2, **caractérisé en ce que** la liaison entre le promoteur d'adhérence et le substrat et/ou la liaison entre la couche fonctionnelle et la couche de support est une liaison covalente.

4. Produit médical selon la revendication 2 ou 3, **caractérisé en ce que** le promoteur d'adhérence est un composé de silicium, en particulier un composé de silane.

5. Produit médical selon l'une des revendications 1 à 4, **caractérisé en ce que** l'alcool glucidique de la couche fonctionnelle, sous sa forme non fonctionnalisée ou déshydratée, comprend au moins un alcool glucidique de formule brute C₆H₁₄O₆.

6. Produit médical selon l'une des revendications 1 à 5, **caractérisé en ce que** le monosaccharide de la couche fonctionnelle, sous la forme non liée à la couche de support ou au substrat, est fonctionnalisé par le biais d'au moins une liaison multiple, en particulier une double liaison, réactive.

7. Produit médical selon la revendication 6, **caractérisé en ce que** la double liaison réactive fait partie d'un groupe (méth)acrylique.

8. Produit médical selon l'une des revendications précédentes, **caractérisé en ce que** le substrat comprend au moins un métal, choisi dans le groupe comprenant le nickel, le titane, le platine, l'iridium, l'or, le cobalt, le chrome, l'aluminium, le fer et/ou un alliage de ceux-ci.

9. Produit médical selon l'une des revendications 1 à 7, **caractérisé en ce que** le substrat comprend au moins une matière plastique, choisie dans le groupe comprenant les polyamides (PA), le polytétrafluoroéthylène (PTFE), le polytétrafluoroéthylène expansé (ePTFE), les acides polylactiques (PLA), le polyester, le polyéther, le polyuréthane, les polyoléfines, les copolymères séquencés en outre correspondants.

10. Produit médical selon l'une des revendications précédentes, **caractérisé en ce qu'**il s'agit d'un produit médical pour l'utilisation endovasculaire.

11. Produit médical selon l'une des revendications 1 à 9, **caractérisé en ce qu'**il s'agit d'un produit médical pour l'utilisation neurovasculaire.

12. Produit médical selon l'une des revendications 1 à 9, **caractérisé en ce qu'**il s'agit d'un produit médical pour l'utilisation cardiovasculaire.

13. Procédé de revêtement d'un produit médical comportant un substrat, avec un revêtement comportant une couche fonctionnelle, qui diminue l'adhérence des thrombocytes, la couche fonctionnelle comprenant au moins un monosaccharide et les monosaccharides étant liés directement ou indirectement au substrat par une fonctionnalisation des monosaccharides et oligomérisant ou polymérisant seulement lors de la liaison au substrat, les monosaccharides, sous leur forme non fonctionnalisée, comprenant un alcool glucidique ou un composé cyclique ou hétérocyclique pouvant être dérivé de celui-ci par déshydratation.
